# EUROPEAN PATENT APPLICATION

(11) **EP 3 848 014 A1**
(43) Date of publication of application: **14.07.2021**
(21) Application number: 19844366.5
(22) Date of filing: 12.06.2019
(51) Int. Cl.: A61K 8/19, A61K 8/60, A61K 8/73, A61Q 19/00, A61Q 19/02

(54) **WATER-SOLUBLE FULLERENE TOPICAL COMPOSITION**

(30) Priority: 02.08.2018 CN 201810871219
(71) Applicant: Beijing Fullcan Biotechnology Co., Ltd, Beijing 101304 (CN); INSTITUTE OF CHEMISTRY, CHINESE ACADEMY OF SCIENCES, Beijing 100190 (CN)
(72) Inventor: LI, Hui, Beijing 101304 (CN); WANG, Chunru, Beijing 101304 (CN); XU, Zhe, Beijing 101304 (CN)
(74) Representative: Maiwald Patent- und Rechtsanwaltsgesellschaft mbH
(86) International application number: PCT/CN2019/090926
(87) International publication number: WO 2020/024703

(57) **Abstract**

A water-soluble fullerene topical composition, a preparation method therefor, and an application. The composition comprises a water-soluble additive, and a fullerene dissolved or dispersed in the water-soluble additive. The composition has a cosmetic effect.

## Description

### Technical Field

The present invention relates to the field of daily use chemicals, and more particularly relates to a topical composition having a cosmetic effect.

### Background Art

In 1985, scientists Kroto, Smalley, Curl and others discovered C60 fullerene while studying carbon in the deep space. In 1996, they won the Nobel Prize in Chemistry for their discovery. A fullerene, diamond and graphite are allotropes of carbon. C60 is a spherical molecule composed of 60 carbon atoms, and consists of 12 pentagons and 20 hexagons. Each carbon atom is bonded with three adjacent carbon atoms by mean of SP² hybridization, and C60 is called CITE because it looks like a football. A series of molecules having cage-like carbon atom cluster structures are collectively referred to as cage Note><Cite><Au fullerene derivatives and heterocyclic fullerenes. C60, C70, C76 and C84 are the normal members.

A large π bond conjugated system composed of a plurality of P orbitals of the fullerene makes it have strong electron acceptability. Due to a particular structure and physico-chemical properties, it has an excellent free radical scavenging capacity, and is called "free radical sponge". In addition, it also shows an excellent antioxygenic property. Therefore, it has been widely concerned in cosmetic materials, biomedicines and other aspects.

By being intercalated into a surfactant, C60 still can scavenge intracellular reactive oxygen, and inhibit the depolarization of mitochondria, the activation of cysteine protease, the exposure of phosphatidylserine on cell membranes and the disintegration of DNA, and C60 protects cells from apoptosis caused by oxidative stress. A C60 liposome complex is 172 times more potent than vitamin C to act as an antioxidant by protecting against UVA damage to skin structures, nuclei and collagenous fibers and by penetrating into human skin tissues, and can be compatible with free radicals.

Professor Fathi Moussa, a longtime researcher on the properties of C60, was the first to study the dissolution of fullerene in olive oil. They fed mice once every two weeks for 10-17 months (four milligrams of fullerene per kilogram of body weight). Mice in a control group died for 17-38 months, while mice in a test group lived for 59-66 months. Fullerene was found to have longevity and health care functions. During the trial, regardless of body weights, there was no abnormal physiological performance, C60 was not found to have toxicity and biological heredity. On the contrary, a certain amount of C60 has the ability to scavenge free radicals, so that animals can avoid the harm caused by excessive free radicals.

As a topical product, there have been patents reported on Fullerene-related products, such as C60-PVP, application number: 200480005008.1 and 200580049615.2. Due to the limitation of a preparation process and composite characteristics of PVP and C60, the content of C60 in the compound is not more than 1%. In addition, PVP has an obvious film-forming property, so excessive application on the skin surface will have a certain hindrance effect on the effect of some cosmetics. C60-squalane has the application number of 201510427642.4, but the solubility of C60 in squalane is limited, and squalane is an oil solution, which limits the use of this series of products. Fullerene nano suspension has the application number of CN201510971343.7, but this ingredient is a large-particle fullerene, which will affect the stability of a formulation, appearance and use comfort when added into the formulation. Moreover, the large-particle fullerene is difficult to play a role in skin repairing, and can only attach to the surface of the skin to act as a barrier.

The fullerene is a nonpolar molecular with strong hydrophobicity, and can only be dissolved in nonpolar organic solvents harmful to organisms, such as methylbenzene, benzene, cyclohexane and chloroform. The hydrophobic nature of the fullerene directly affects the play of its biological activity. In order to better promote absorption, the fullerene is prepared into a solution which is easy to be absorbed by skin quickly and is in a molecular state. However, the ingredients for preparing the fullerene into a skin-friendly type have not been studied. Raw materials of a water-soluble fullerene obtained by the present invention are all of cluster properties, uniform and transparent in an aqueous solution, stable and non-precipitating. The selected excipients do not have surfactant properties, will not stimulate the skin, and have good skin repairing and moisturizing effects. The composition contains a high content of fullerene and has better efficacy. The composition containing fullerene of the present invention ensures that the fullerene is well dispersed in the selected solvent, and the obtained the composition containing fullerene is stable in properties and can be applied to any topical formulation. The fullerene in the composition of the present invention is in a dissolved state, and as an ingredient of a topical product, it can better act on a skin.

### Summary of the Invention

A purpose of the present invention is to provide a fullerene composition, wherein a fullerene in the composition is in a dissolved state, can be absorbed quickly, and has moisturizing, moisture locking, antioxidation and other beauty effects.

In an aspect, the present invention provides a fullerene composition which contains a carbohydrate additive and a fullerene dissolved or dispersed in the carbohydrate additive.

In an aspect, the present invention provides a fullerene dispersion system which contains a fullerene and a carbohydrate additive, and is characterized in that particle sizes of the dispersion system are mainly between 690 nm and 710 nm, for example, more than 50%, such as more than 60%, 70%, 80%, 90%, 95% and 99%, of the particle sizes are between 690 nm and 710 nm.

In an aspect, the present invention provides a fullerene dispersion system which contains a fullerene and a carbohydrate additive, and is characterized in that more than 50%, such as more than 60%, 70%, 80%, 90%, 95% and 99%, of particle sizes in the dispersion system are between 690 nm and 700 nm.

In an aspect, the present invention provides a cosmetic or drug delivery system and/or sustained-release system, which is characterized by including any one of the above-mentioned compositions or dispersion systems.

In an aspect, the present invention provides a preparation method for a fullerene composition or a dispersion system, which is characterized by including the following steps: (1) uniformly mixing a fullerene powder and a carbohydrate powder; (2) grinding the mixture; (3) dissolving with water; (4) centrifuging at a high-speed; and (5) filtering; optionally, the method further includes the steps of (6) concentrating; and (7) drying; wherein optionally, a finished product obtained at step (7) may be further dissolved with water or reconstituted as a required solution; optionally, during the grinding, a dissolution degree is monitored by means of ultraviolet absorbance; optionally, the grinding is performed by use of a ball grinder, a colloid grinder, a sand grinder or a homogenizer; and optionally, a G4 sand core funnel is used for filtering.

In an aspect, the present invention provides a fullerene composition, which is characterized by being prepared by the method of claim 4.

According to any one of the above-mentioned aspects, the carbohydrate additive is selected from monosaccharide, disaccharide, oligosaccharide, polysaccharide and a saccharide amine; optionally, the monosaccharide is selected from fructose, galactose and glucose; optionally, the disaccharide is selected from saccharose, lactose, maltose, melibiose and cellobiose; optionally, the polysaccharide is selected from cellulose and a hyaluronic acid; optionally, the saccharide amine is selected from acetylchitosamine, lactamide and glucosamine; optionally, the cellulose is, for example, hydroxyethyl cellulose, ethyl cellulose, microcrystalline cellulose, hydroxypropyl methyl cellulose and methyl cellulose; optionally, the hyaluronic acid is, for example, hyaluronic acid, sodium hyaluronate, potassium hyaluronate, hydrolyzed hyaluronic acid and hydrolyzed sodium hyaluronate, preferably, the carbohydrate additive is at least one of hydroxyethyl cellulose, sodium hyaluronate and glucose, more preferably, the carbohydrate additive is hydroxyethyl cellulose, sodium hyaluronate and glucose; optionally, a weight ratio of the carbohydrate to the fullerene is (20-200): 1, preferably, is (50-200): 1, further preferably, is (70-150): 1, for example, about 50: 1, 60: 1, 70: 1, 80: 1, 90: 1, 100: 1, 110: 1, 120: 1, 130: 1, 140: 1, 150: 1, 160: 1, 170: 1, 180: 1, 190: 1 and 200: 1. Unless otherwise defined, the term "about" in context covers a range from 10% less than a value defined to 10% more than the value defined, preferably, from 5% less than the value defined to 5% more than the value defined. For example about 10, includes but is not limited to 9, 9.1, 9.2, 9.3, 9.4, 9.5 or 10.1, 10.2, 10.3, 10.4 and 10.5. According to any one of the above-mentioned aspects, the fullerene is at least one of a hollow fullerene, a metallic fullerene, a heterocyclic fullerene and an endohedral fullerene, includes but is not limited to any one or a mixture of fullerene C₂ₙ, M@C₂ₙ, M₂@C₂ₙ, MA@C₂ₙ, M₃N@C₂ₙ, M₂C₂@C₂ₙ, M₂S@C₂ₙ, M₂O@C₂ₙ and M_{X}A₃₋ₓN@C₂ₙ, wherein, M and A are both metallic elements, the M and the A are both selected from any one of Sc, Y and lanthanide metallic elements; and are 00 family elements among the elements of 30 families, preferably, the fullerene is any one or a mixture of C60, C70, C76, C84, and Gd@C82 and derivatives thereof, and further preferably, the fullerene is C60.

According to any one of the above-mentioned aspects, the composition is characterized by being composed of hydroxyethyl cellulose, sodium hyaluronate, glucose and fullerene, optionally, a ratio of the composition is (10-50): (5-20): (30-80): (0.1-10), for example, the ratio is 40: 10: 49: 1, 30: 20: 49: 1, 20: 10: 69: 1, 30: 5: 64: 1, 30: 10: 59: 0.1, preferably, the ratio is 30: 10: 59: 1; optionally, the composition further contains other organic solvents and/or water serving as a solvent.

An application of the composition or dispersion system according to any one of the above-mentioned aspects in the preparation of a beauty product or a cosmetic.

An application of the composition or dispersion system according to any one of the above-mentioned aspects in the manufacturing of a beauty or make-up tool.

According to any one of the above-mentioned aspects, the present invention provides a beauty method, which includes the step of applying an active amount of the composition of the present invention or a drug containing the composition of the present invention to a subject, optionally, the beauty effect includes at least one of whitening and spot-removing, wrinkle-reducing, anti-acne, and anti-hair loss and hair strengthening effects.

According to any one of the above-mentioned aspects, the composition of the present invention further can be used together with other excipients.

According to any one of the above-mentioned aspects, the composition of the present invention can be used together with a surfactant.

According to any one of the above-mentioned aspects, the composition of the present invention can be added into a cosmetic or drug delivery system and/or sustained-release system.

The composition of the present invention may be in a form of a liquid preparation, such as a water-based suspension or a solution, or further may be a dry product that can be combined with water or other suitable carriers before use. Such liquid preparations may contain conventional additives, such as suspending agents, such as sorbitol, methyl cellulose, gelatin, hydroxyethyl cellulose, carboxymethyl cellulose, an aluminum stearate gel or a hydrogenated edible fat; and the solution is generally prepared by dissolving an active substance in a carrier, then filtering and sterilizing before being placed in a suitable smaller bottle or ampoule bottle, and sealing.

In an aspect, the present invention provides a method for preparing an aqueous solution by using a fullerene composition, including: mixing the fullerene composition of the present invention and water according to a required ratio, uniformly stirring, homogenizing, adding a preservative and a fragrance to obtain the aqueous solution.

The composition of the present invention further may be prepared in a form of an emulsion, including conventional emulsifiers, auxiliary emulsifiers, surfactants, etc.

In an aspect, the present invention provides a method for preparing an emulsion by using a fullerene composition, including: mixing the fullerene composition and an emulsifier according to a ratio, homogenizing to form a milky or paste mixture, and adding a preservative and a fragrance to obtain the emulsion.

According to any one of the above-mentioned aspects, the beauty product or the cosmetic is used for whitening and spot-removing, wrinkle-reducing, anti-acne, haze-proofing, anti-hair loss and hair strengthening, etc.

### Brief Description of the Drawings

Figure 1 shows dynamic light scattering experimental results of fullerene solutions, wherein, figure 1A is sodium hyaluronate-fullerene, figure 1B is hydroxyethyl cellulose-fullerene, figure 1C is glucose-fullerene, and figure 1D is hyaluronic acid -hydroxyethyl cellulose-glucose-fullerene;
Figure 2 shows free radical scavenging results of compositions of the present invention, wherein, figure 2A is a black test, figure 2B is a 5 ppm (the content of fullerene) test group, figure 2C is a 25 ppm (the content of fullerene) test group, and figure 2D is 25 ppm water-soluble vitamin C;
Figure 3 shows a mean MMV value of a composition of the present invention;
Figure 4 shows a mean TEWL value of a composition of the present invention;
Figure 5 shows an effect of a composition of the present invention in alleviating acne inflammation;
Figure 6 shows an effect of a composition of the present invention in reducing an erythema area on an acne skin (a percentage of the erythema area);
Figure 7 shows an effect of a composition of the present invention in reducing an erythema area on an acne skin (a change rate of the erythema area);
Figure 8 shows an effect of a composition of the present invention in reducing color spots;
Figure 9 shows a haze-proofing experiment of a composition of the present invention;

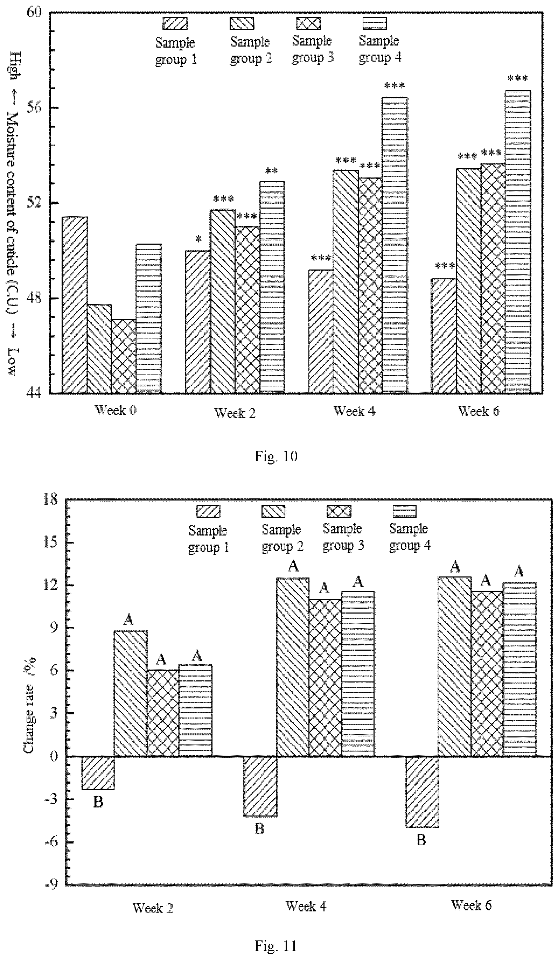

Figure 10 shows a change trend of a moisture content of local cuticle (a comparison of all time points, *p<0.05, **p<0.01, ***p<0.001);
Figure 11 shows a change rate of a moisture content of local cuticle (an inter-group significance analysis, a significant difference exits between different letters);

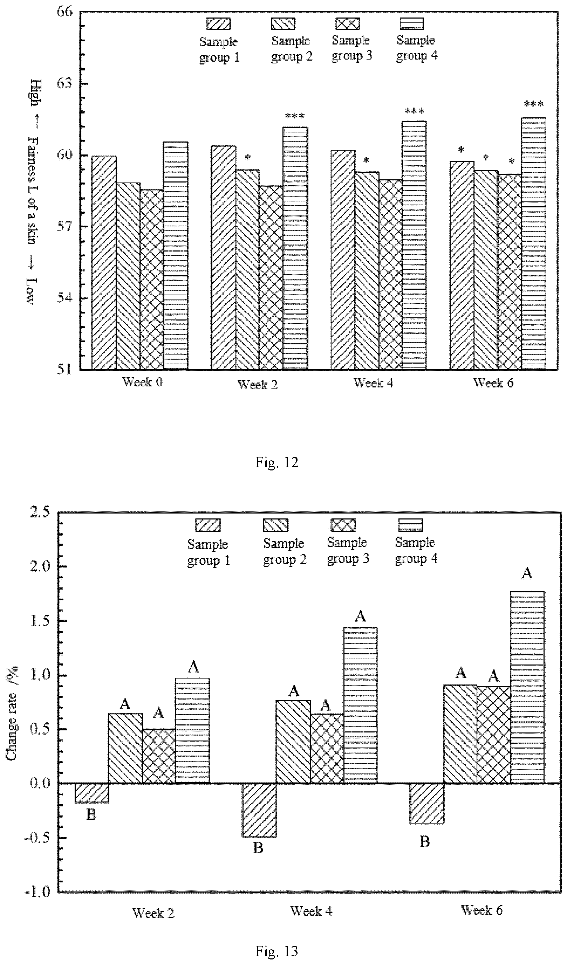

Figure 12 shows a change trend of a fairness L of a local skin (a comparison of all time points, *p<0.05, **p<0.01, ***p<0.001);
Figure 13 shows a change rate of a fairness L of a local skin (an inter-group significance analysis, a significant difference exits between different letters);

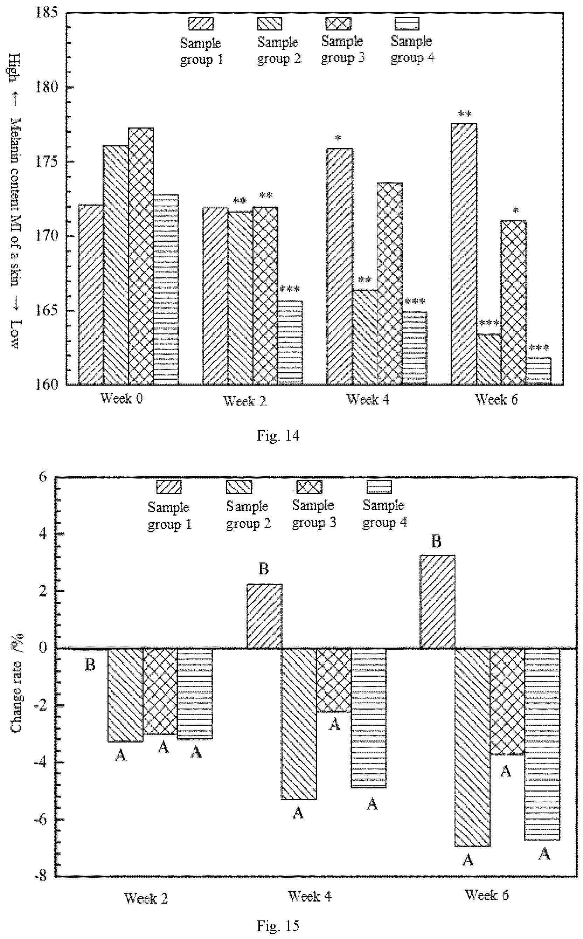

Figure 14 shows a change trend of a melanin content MI of a local skin (a comparison of all time points, *p<0.05, **p<0.01, ***p<0.001);
Figure 15 shows a change rate of a melanin content MI of a local skin (an inter-group significance analysis, a significant difference exits between different letters);

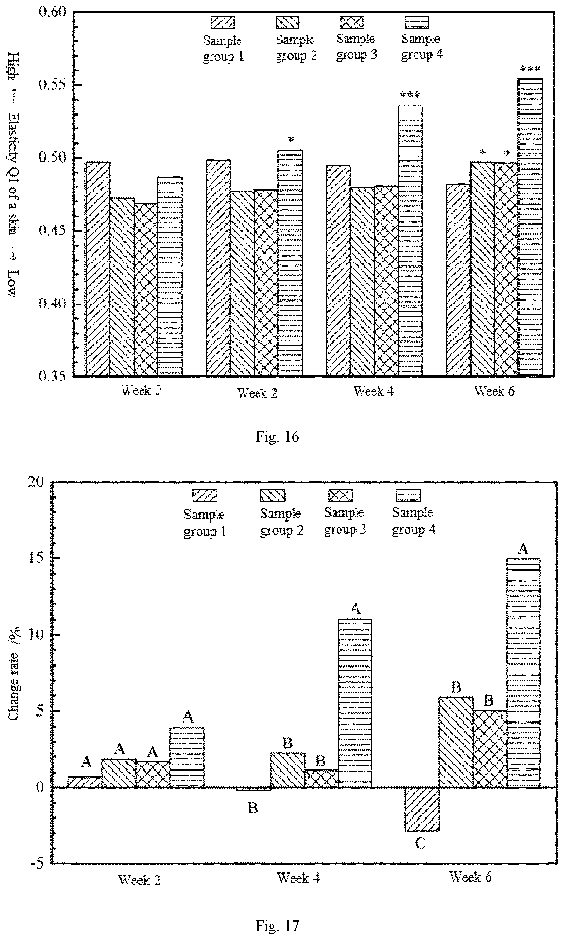

Figure 16 shows a change trend of elasticity Q1 of a local skin (a comparison of all time points, *p<0.05, **p<0.01, ***p<0.001);
Figure 17 shows a change rate of elasticity Q1 of a local skin (an inter-group significance analysis, a significant difference exits between different letters);

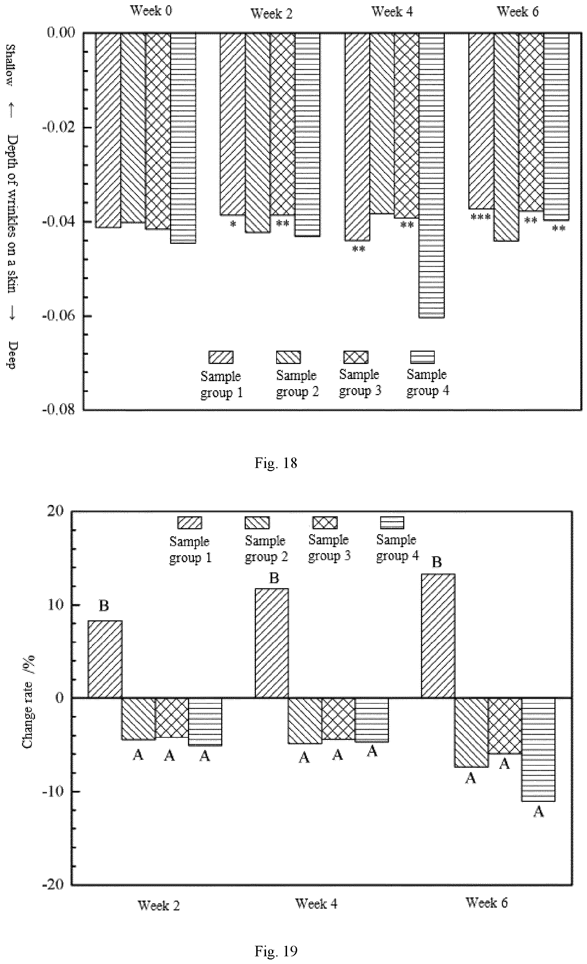

Figure 18 shows a change trend of a depth of wrinkles on a local skin (a comparison of all time points, *p<0.05, **p<0.01, ***p<0.001); and
Figure 19 shows a change rate of a depth of wrinkles on a local skin (an inter-group significance analysis, a significant difference exits between different letters);

### Detailed Description of the Invention

Specific implementation modes of the present invention will be described below in conjunction with the accompanying drawings, but it should be understood that the scope of protection of the present invention is not limited to the specific implementation modes.

Unless otherwise defined, in the whole description and claims, the terms "include" or its synonyms such as "comprise" or "have" should be understood that the elements and parts stated are included, but other elements or parts are not excluded.

In an implementation, the present invention provides a fullerene composition which contains a carbohydrate additive and a fullerene dissolved or dispersed in the carbohydrate additive, optionally, a concentration of the fullerene is 0.01-5 mg/ml, preferably, 0.05-3 mg/ml, more preferably, 0.1-2 mg/ml, further preferably, 1 mg/ml.

In an implementation, the composition or dispersion system is composed of hydroxyethyl cellulose, sodium hyaluronate, glucose and fullerene, optionally, a weight ratio of the composition is (10-50): (5-20): (30-80): (0.1-10), for example, the ratio is 40: 10: 49: 1, 30: 20: 49: 1, 20: 10: 69: 1, 30: 5: 64: 1, and 30: 10: 59: 0.1, preferably, the ratio is 30: 10: 59: 1.

In an implementation, the composition may further contain other organic solvents and/or water serving as a solvent, for example, ingredients of the composition are hydroxyethyl cellulose, sodium hyaluronate, glucose, fullerene, 1-3 butanediol and water, and a weight ratio is 6: 2: 11.8: 0.02: 75: 5.18.

In an implementation, the present invention provides a preparation method for a fullerene composition or a dispersion system, which is characterized by including the following steps that: (1) a fullerene powder and a carbohydrate additive are uniformly mixed; (2) compounding is performed by means of ball grinding; (3) dissolution is performed: the prepared fullerene composite material is completely dissolved with deionized water; (4) centrifugation is performed: the solution obtained after dissolution is centrifugated at a high-speed of 8500 r/min for 5 min to remove undissolved large particles in the solution; (5) filtration is performed: fine precipitates in the solution are filtered out by using a G4 sand core funnel; (6) concentration is performed: most of water in the obtained filtrate is removed by means of vacuum rotary evaporation or a micro-filtration membrane; and (7) drying is performed: the water is removed by means of freezing drying or spray drying to obtain a water-soluble fullerene powder.

In an implementation, the present invention provides a fullerene composition, which is characterized by further containing at least one antioxidant in addition to the carbohydrate additive and the fullerene, wherein the antioxidant is selected from at least one of superoxide dismutase, mannitol, histidine, tryptophan, bilirubin, quercetin, quercetin, polyphenol, procyanidin, tocotrienol, catechin, derivatives of catechin, rutin and its derivatives, gallic acid and its derivatives, ubiquinone, astaxanthin, carotene, other carotenoids and their derivatives as well as their salts, vitamin B group and their derivatives as well as their salts, vitamin D group and their derivatives as well as their salts, vitamin E group and their derivatives as well as their salts, butylated hydroxytoluene and butylated hydroxyanisole.

In an implementation, the present invention provides a fullerene composition, which is characterized by further containing at least one active ingredient in addition to the carbohydrate additive and the fullerene, wherein the active ingredient includes but is not limited to: a matrix metalloproteinase inhibitor, an irritant or an inhibitor for melanogenesis, a brightener or a decolorizer, a pigmentogenesis agent, a sun-screening agent, an anti-aging agent, NO-synthase inhibitor, an antioxidant, a free radical scavenging agent and/or an anti-air pollution agent, an anti-saccharification agent, an emulsifier, an emollient, an organic solvent, a liquid propellant, a skin modifier such as a wetting agent, a substance for keeping moisture, alpha hydroxy acid, beta hydroxy acid, a moisturizing agent, an epidermis hydrolase, a vitamin, a pigment or a coloring agent, a dye, a gel polymer, a thickener, a surfactant, a softener, an anti-wrinkle agent, an agentia being capable of reducing or treating eye-bag, a peeling, an antimicrobial, a fungicide, a fungistat, a bactericide, a bacteriostatic agent, an irritant for dermis or epidermis macromolecular synthesis and/or an agentia being capable of stopping or inhibiting dermis or epidermis macromolecular degradation, an irritant for collagen synthesis, an irritant for elastin synthesis, an irritant for core proteoglycan synthesis, an irritant for laminin synthesis, an irritant for defensin synthesis, an irritant for molecular chaperone synthesis, an irritant for aquaporin synthesis, an irritant for hyaluronic acid synthesis, an irritant for synthesis of components of lipid and cuticle, an irritant for ceramide synthesis, an inhibitor for collagen degradation, an inhibitor for elastin degradation, an irritant for fibroblast proliferation, an irritant for keratinocyte proliferation, an irritant for adipocyte proliferation, an irritant for melanocyte proliferation, an irritant for keratinocyte differentiation, an irritant for adipocyte differentiation, an acetylcholine esterase inhibitor, a skin relaxant, an irritant for glycosaminoglycan synthesis, a DNA repairing agent, a DNA protective agent, an antipruritic, an agentia for treating and/or caring a sensitive skin, a curing agent, an anti-vergeture agent, an astringent, an agentia for adjusting sebum production, an irritant for lipodieresis, an anti-cellulitis agent, an irritant for healing, an adjuvant for healing, in irritant for epithele regeneration, an adjuvant for epithele regeneration, a growth factor of cell factors, a depressant, anti-inflammatory agent, an agentia acting on capillary circulation and/or microcirculation, in irritant for angiogenesis, an inhibitor for vascular permeability, an agentia acting on cell metabolism, an agentia for improving dermis-epidermis joint, an agentia prompting hair growth, an agentia inhibiting or delaying hair growth, a preservative, a fragrance, a chelating agent, a plant extract, essential oil, a marine extract, an agentia from a biological fermentation process, a mineral salt, a cell extract, a sunscreen (an organic or mineral light protective agent having an effect of blocking ultraviolet A and/or ultraviolet B), or their mixtures.

In an implementation, the present invention provides an application of a fullerene composition in preparation of a beauty product or a cosmetic.

In an implementation, a form of the beauty product or cosmetic includes but is not limited to any form of a solution, a suspension, an opacifier, a paste, a gel, a cream, an emulsion, a powder, a soap, a cleaning agent containing a surfactant, oil, a powdery foundation cream, an emulsion, a foundation cream, a wax-based foundation cream, a spray, a liniment, a cataplasm and a poultice, such as a cream, a complex emulsion, an anhydrous composition, a water dispersible agent, oil, an emulsion, a balm, a foam, a cleaning liquid, a gel, a creamy gel, a water alcohol solution, a water diol solution, a liniment, a saline solution, a soap, a shampoo, a modifier, a slurry, an oleamen, a mousse, an ointment, a powder, a stick agent, a pen agent, a spray, an aerosol, a capsule, a gelatin capsule, a tablet, a sugar-coated tablet, a powder, a particle form, a chewing gum, a solution, a suspension, an emulsion, a syrup, a polysaccharide film, a jelly, a gelatin, an ointment, a cream, a softening lotion, a nourishing lotion, a mask, a serum, a hair restorer, a shampoo, a rinsing agent, a hair conditioner, a hair care agent, a gel, a skin emulsion, a skin softening agent, a lotion, an astringent, an emulsion, a milky emulsion, a moisturizing emulsion, a nourishing emulsion, a massage cream, a nourishing cream, a moisturizing cream, a hand cream, a foundation cream, a nourishing serum, a sunscreen cream, a soap, a cleaning foam, a cleaning milk, a cleaning cream, a body lotion and a body cleanser, an eye-bag remover, a make-up foundation, a make-up remover, a make-up cleaning milk, an eyeshadow, a lipstick, a lip gloss and a lip powder; an effect of the beauty product and cosmetic includes but is not limited to skin whitening, improving pigementation, treating acnes, improving wrinkles, improving roughness of skin, improving an oily skin, improving a dry skin, shrinking pores, treating scars, treating redness of skin, treating hair-loss, promoting hair growth, treating burn injuries, sterilizing skin, eliminating mites on a skin, improving dimples on a skin, resisting acnes, and proofing haze.

In an implementation, the present invention provides an application of a fullerene composition in manufacturing a beauty or make-up tool, wherein the tool includes but is not limited to an antiseptic prep pad, an antiseptic cotton swab, a disinfection package, a medical dressing wipe, a care solution, a cleaning liquid, a dressing, a liquid dressing, a bandage; the application may be attaching the composition of the present invention onto a specific material, and the material includes but is not limited to a cotton bub, a wipe, a towel, a paper tissue, a gauze, a cotton swab, a nonwoven, a high polymer material, a medical dressing, a non-stick gauze for plastic models, a petrolatum gauze, a semi-permeable membrane and a foam excipient.

In an implementation, the composition of the present invention further can be used together with other excipients, wherein the excipient includes hyaluronic acid, lipids, a surfactant, a preservative, a fragrance, an adhesive, a thickener, a complexant and a PH modifier.

In an implementation, the composition of the present invention can be used together with a surfactant, wherein the surfactant is selected from at least one of an anionic surfactant, a cationic surfactant, an ampholytic surfactant, a lipophilic nonionic surfactant, a hydrophilic nonionic surfactant, and a natural surfactant.

In an implementation, the anionic surfactant may be, for example, at least one of a fatty acid soap such as a soap raw material, sodium laurate and sodium palmitate, an advanced alkyl sulfate salt such as sodium lauryl sulfate and potassium lauryl sulfate, an alkyl ether sulfate salt such as polyoxyethylene (POE)-triethanolamine lauryl sulfate and POE-sodium lauryl sulfate, N-acyl sarcosine such as sodium lauroyl sarcosinate, advanced fatty acid amide sulfonate such as N-tetradecanoyl-N-sodium methyl taurate, sodium coconut oil fatty acid methyl taurate and sodium lauryl methyl taurate, a phosphate salt such as POE- sodium oleyl ether phosphate, POE-stearyl ether phosphate, sulfonic succinate such as di-2-sodium ethylhexyl sulfosuccinate, sodium monolauroyl monoethanolamide polyoxyethylene sulfosuccinate, sodium lauryl polypropylene glycol sulfosuccinate, alkyl benzenesulfonate such as linear sodium dodecyl benzenesulfonate, linear triethanolamine dodecyl benzenesulfonate, linear dodecyl benzenesulfonate, N-acyl glutamate such as N-monosodium lauroyl glutamate, N-disodium stearyl glutamate and N-tetradecanoyl-L-monosodium glutamate, advanced fatty acid ester sulfate such as hardened sodium coconut oil fatty acid glycerol sulfate, sulfated oil such as sulfonated castor oil, POE-alkyl ether carboxylic acid, POE-alkyl allyl ether carboxylate, alpha olefin sulfonate, advanced fatty acid ester sulphonate, secondary alcohol sulfate, advanced fatty acid alkyl alcohol acylamide sulfate, sodium lauryl monoethanolamide succinate, N- di-triethanolamine palmitoyl aspartate and sodium caseinate.

In an implementation, the cationic surfactant may be, for example, at least one of alkyl trimethyl ammonium salt such as stearyl trimethyl ammonium chloride and lauryl trimethyl ammonium chloride, distearyl dimethyl ammonium chloride dialkyl dimethyl ammonium salt, an alkyl piperidinium salt such as chlorinated poly(N,N'-dimethy-3,5-methylene piperidinium) and chlorinated cetyl piperidinium, an alkyl quaternary ammonium salt, an alkyl dimethyl benzyl ammonium salt, an alkyl isoquinolinium salt, a dialkyl morpholinium salt, POE-alkylamine, an alkyl ammonium salt, derivatives of polyamine fatty acid, derivatives of pentyl alcohol fatty acid, benzalkonium chloride and benzethonium chloride.

In an implementation, the ampholytic surfactant may be, for example, at least one of an imidazoline ampholytic surfactant such as 2-undecyl-N,N,N-(hydroxyethyl carboxymethyl)-2-sodium imidazoline and 2-coco-2-imidazoline hydroxide-1-carboxyethoxyl disodium salt, and an betaine ampholytic surfactant such as 2-heptadecyl-N-carboxymethyl-N-hydroxyethyl imidazolonium betaine, lauryl dimethyl glycine betaine, alkyl betaine, amide betaine and sulfonic betaine.

In an implementation, the lipophilic nonionic surfactant may be, for example, at least one of sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, peta-2-ethylhexanoic diglycerol sorbitan and tetra-2-ethylhexanoic diglycerol sorbitan, fatty acid glycerides such as cottonseed oil fatty acid monoglyceride, erucidic monoglyceride, sesquioleate glyceride, glyceryl monostearate, α,α'-oleic pyroglutamic glyceride and glyceryl monostearate, polyglycerol fatty acid esters such as diglycerol monisostearate and diglycerol diisostearate, propanediol fatty acid esters such as propanediol monostearate, derivatives of hardened castor oil and glycerol alkyl ether.

In an implementation, the hydrophilic nonionic surfactant may be, for example, at least one of POE-sorbitan fatty acid esters such as POE-sorbitan monooleate, POE-sorbitan monostearate, POE-sorbitan monooleate and POE-sorbitan tetraoleate, POE-sorbitol fatty acid esters such as POE-sorbitol monolaurate, POE-sorbitol monooleate, POE-sorbitol pentaoleate and POE-sorbitol monostearate, POE-glycerin fatty acid esters such as glycerin monostearate, POE-glycerin monoisostearate and POE-glycerin triisostearate, POE-fatty acid esters such as POE-monooleate, POE-distearate, POE-monodioleate and ethylene glycol distearate, POE-alkyl ethers such as POE-lauryl ether, POE-oleyl ether, POE-stearyl ether, POE-behenate ether, POE-2-octyl dodecyl ether and POE-cholestanol ether, POE.POP-alkyl ethers such as POE.POP-cetyl ether, POE.POP-bi(decyl tetradecyl ether), POE.POP-monobutyl ether, POE.POP-hydrogenated lanolin and POE.POP-glycerin ether, derivatives of POE-castor oil/hardened castor oil such as POE-castor oil, POE-hardened castor oil, POE-hardened castor oil monoisostearate, POE-hardened castor oil triisostearate, POE-hardened castor oil monopyroglutamate monoisostearate diester and POE-hardened castor oil maleate, derivatives of POE-beeswax•lanolin such as POE-sorbitol beeswax, alkanolamide such as coconut oil fatty acid diethanolamide, lauroyl monoethanolamide, fatty acid isopropanolamide, POE-propanediol fatty acid ester, POE-alkylamine, POE-fatty acid amide, sucrose fatty acid ester, condensates of POE-nonylphenylformaldehyde, alkyl ethoxy dimethyl amine oxide and trioleyl phosphate.

In an implementation, the natural surfactant may be, for example, at least one of lecithin such as soy phosphatidylcholine, hydrogenated soy phosphatidylcholine, egg yolk lecithin, hydrogenated egg yolk lecithin, or soy saporin.

In an implementation, the composition of the present invention may be added into a cosmetic or drug delivery system and/or sustained-release system, wherein the cosmetic or drug delivery system and/or sustained-release system is selected from combinations composed of liposome, mixed liposome, millimetric microcapsule, microcapsule, nanometric microcapsule, sponge, cyclodextrin, niosome, micelle, mixed surfactant micelle, phospholipid- surfactant mixed micelle, millimetric microsphere, microsphere, nanometric microsphere, lipid microsphere, microemulsion, nanometric emulsion, millimetric particle, particle, nanoparticle and solid lipid nanoparticle.

In an implementation, the composition of the present invention may be in a form of a liquid preparation such as an aqueous suspension or solution, or may be a dry product that can be combined with water or other suitable carrier before use. Such liquid preparations may contain conventional additives, such as a suspending agent, for example, sorbitol, methyl cellulose, gelatin, hydroxyethyl cellulose, carboxymethyl cellulose, an aluminum stearate gel or a hydrogenated edible fat; and the solution is generally prepared by dissolving an active substance in a carrier, filtering and sterilizing the mixture before being placed into a suitable small bottle or ampoule bottle, and sealing.

In an implementation, the present invention provides a method for preparing an aqueous solution by using a fullerene composition, which includes that: the fullerene composition of the present invention is mixed with water according to a ratio and uniformly stirred, well homogenized, and added with a preservative and a fragrance to obtain the aqueous solution.

In an implementation, the composition of the present invention is prepared into a form of an emulsion including a conventional emulsifier, an auxiliary emulsifier, a surfactant, etc.

In an implementation, the present invention provides a method for preparing an emulsion by using a fullerene composition, which includes that: the fullerene composition of the present invention is mixed with an emulsifier according to a ratio and then homogenized to form a milky or paste mixture, and added with a preservative and a fragrance to obtain the emulsion.

In an implementation, the present invention provides an application of the composition or dispersion system of the present invention in preparation of a beauty product or a cosmetic.

In an implementation, the present invention provides an application of the composition or dispersion system of the present invention in manufacturing a beauty or make-up tool.

### Embodiments

The present invention will be further explained by referring to embodiments. The description of the specific exemplary embodiments is used for the purposes of explanation and illustration. The description is not intended to limit the present invention to the specific embodiments disclosed, and apparently, various changes and transformations can be made according to the enlightening of the description of the present invention. The selection and description of the exemplary embodiments are used for explaining the specific principle and practical applications of the present invention, so that the person skilled in the art can implement and apply the various exemplary embodiments, as well as various selection and changes of the present application.

### Embodiment 1: Compounding of hydroxyethyl cellulose, sodium hyaluronate, glucose, and fullerene

### 1) Compounding of hydroxyethyl cellulose and fullerene by means of ball grinding

A. Samples were taken at different time points of different ball grinding times. 0.1 g of the hydroxyethyl cellulose: fullerene composition was weighted, slowly added into 80 ml of deionized water, and dissolved and stirred for 30 min.

| Ball grinding time/h | Dissolution velocity | Color of solution | Processing manner for solution | Ultraviolet detection A |
|---|---|---|---|---|
| 4 | No significant difference | With the increase of ball grinding time, the color of the solution deepened gradually | 8500r/min,5min | 0.4708 |
| 6 | | | | 0.6632 |
| 8 | | | | 0.8491 |
| 10 | | | | 0.8925 |
| 12 | | | | 0.8006 |

Conclusion: under the condition of the same feed ratio, with the increase of the ball grinding time, the ultraviolet detection absorbance A gradually increases, which indicates that the content of the fullerene in the water-soluble composition increases gradually, and a compounding rate gradually increases, however, when the ball grinding time reaches 12 h, the content of the fullerene is lower than that when the ball grinding time reaches 10 h, which indicates that too long ball grinding time causes the decrease of the solubility of the water-soluble composition, accordingly, the content of the fullerene in the aqueous solution decreases, after being centrifugated at a high speed, the fullerene can be completely dissolved without precipitation when the ball grinding time is 10 h.
B. The same ball grinding time (10h) and different feed ratios were adopted. 0.1 g of the composition was weighted, slowly added into 80 ml of deionized water, and dissolved and stirred for 30 min.

| Mass ratio of hydroxyethyl cellulose: fullerene | Dissolution velocity | Color of solution | Processing manner for solution | Ultraviolet detection A |
|---|---|---|---|---|
| 50: 1 | With the increase of the mass of the fullerene, the solubility decreased gradually | With the increase of the mass of the fullerene, the color of the solution deepened gradually | 8500r/min, 5min | 0.2744 |
| 40: 1 | | | | 0.3154 |
| 30: 1 | | | | 0.8699 |
| 20: 1 | | | | 0.6502 |

Conclusion: under the condition of the same ball grinding time and different mass ratios, with the increase of the mass of the fullerene, the color of the compound gradually deepens; the solubility gradually decreases; and precipitates gradually increase after centrifugation.

### 2) Compounding of sodium hyaluronate and fullerene by means of ball grinding

A. Samples were taken at different time points of different ball grinding times. 0.1 g of the sodium hyaluronate: fullerene composition was weighted, slowly added into 80 ml of deionized water, and dissolved and stirred for 30 min.

| Ball grinding time/h | Dissolution velocity | Color of solution | Processing manner for solution | Ultraviolet detection A |
|---|---|---|---|---|
| 4 | With the increase of the ball grinding time, the dissolution velocity increased gradually | With the increase of the ball grinding time, the color of the solution deepened gradually | Filtration with a G4 sand core funnel (Fine precipitates were filtered out with the funnel of 3-4 um) | \ |
| 6 | | | | 0.2875 |
| 8 | | | | 0.4171 |
| 10 | | | | 0.5497 |
| 12 | | | | 0.5557 |

Conclusion: under the condition of the same feed ratio, with the increase of the ball grinding time, the ultraviolet detection absorbance A gradually increases, which indicates that the content of the fullerene in the water-soluble composition gradually increases and a compounding rate gradually increases, when the ball grinding time reaches 12 h, the content of the fullerene is slightly greater than that when the ball grinding time reaches 10 h, after being centrifugated at a high speed and filtered by using the G4 sand core funnel, a water-soluble fullerene material is obtained.
B. The same ball grinding time (12h) and different feed ratios were adopted. 0.1 g of the composition was weighted, slowly added into 80 ml of deionized water, and dissolved and stirred for 30 min.

| Mass ratio of sodium hyaluronate: fullerene | High-speed centrifugation | Filtration with a G4 sand core funnel |
|---|---|---|
| 50: 1 | With the increase of the content of the fullerene, the amount of precipitates increased | The solution was basically colorless after filtration |
| 40: 1 | | |
| 30: 1 | | |
| 20: 1 | | |

Conclusion: under the condition of the same ball grinding time and different feed ratios, with the increase of the mass of the fullerene, the amount of the precipitates after centrifugation gradually increases, the color of the supernatant is lighter; after being filtered with the G4 sand core, the filtrate is basically colorless, which indicates that the solubility of a finally obtained water-soluble material decreases with the increase of the mass of the fullerene.

### 3) Compounding of glucose and fullerene by means of ball grinding

A. Samples were taken at different time points of different ball milling times. 0.1 g of the glucose: fullerene composition was weighted, slowly added into 80 ml of deionized water, and dissolved and stirred for 30 min.

| Ball grinding time/h | Dissolution velocity | Color of solution | Processing manner for solution | Ultraviolet detection A |
|---|---|---|---|---|
| 4 | With the increase of the ball grinding time, the dissolution velocity increased gradually | With the increase of the ball grinding time, the color of the solution deepened gradually | Filtration with a G4 sand core funnel (Fine precipitates are filtered out by using the funnel of 3-4 um) | \ |
| 6 | | | | 0.2875 |
| 8 | | | | 0.2171 |
| 10 | | | | 0.3497 |
| 12 | | | | 0.3557 |

Conclusion: under the condition of the same feed ratio, with the increase of the ball grinding time, the ultraviolet detection absorbance A gradually increases, which indicates that the content of the fullerene in the water-soluble composition gradually increases and a compounding rate gradually increases, when the ball grinding time reaches 12 h, the content of the fullerene is slightly greater than that when the ball grinding time reaches 10 h, after being centrifugated a high speed and filtered by using the G4 sand core funnel, a water-soluble fullerene material is obtained.
B. The same ball grinding time (12h) and different feed ratios were adopted. 0.1 g of the composition was weighted, slowly added into 80 ml of deionized water, and dissolved and stirred for 30 min.

| Mass ratio of glucose: fullerene | High-speed centrifugation | Filtration with a G4 sand core funnel |
|---|---|---|
| 50: 1 | With the increase of the content of the fullerene, the amount of precipitates increased | The solution was basically colorless after filtration |
| 40: 1 | | |
| 30: 1 | | |
| 20: 1 | | |

Conclusion: under the condition of the same ball grinding time and different feed ratios, with the increase of the mass of the fullerene, the amount of the precipitates after centrifugation increases gradually, the color of the supernatant is lighter; after being filtered by the G4 sand core, the filtrate is basically colorless, which indicates that the solubility of a finally obtained water-soluble material decreases with the increase of the mass of the fullerene.

### 4) A preparation process of a compound water-soluble fullerene material

Preparation manner: hydroxyethyl cellulose, sodium hyaluronate and glucose were mixed, and then compounded with a fullerene powder
Ball grinding time: 10 h
Processing for a water-soluble fullerene material
Dissolution: a prepared fullerene compound material was completely dissolved with deionized water
Centrifugation: a solution obtained after dissolution was centrifugated at a high speed of 8500 r/min for 5 min, and undissolved large particles in the solution were removed;
Filtration: fine precipitates in the solution were filtered out by using a G4 sand core funnel; Concentration: most of water in an obtained filtrate was removed by means of vacuum rotary evaporation or a micro-filtration membrane;
Drying: the water in the solution was removed by means of freeze drying or spray drying to obtain a water-soluble fullerene powder.

### Embodiment 3: Measurement of particle sizes of materials in an aqueous solution by means of dynamic light scattering

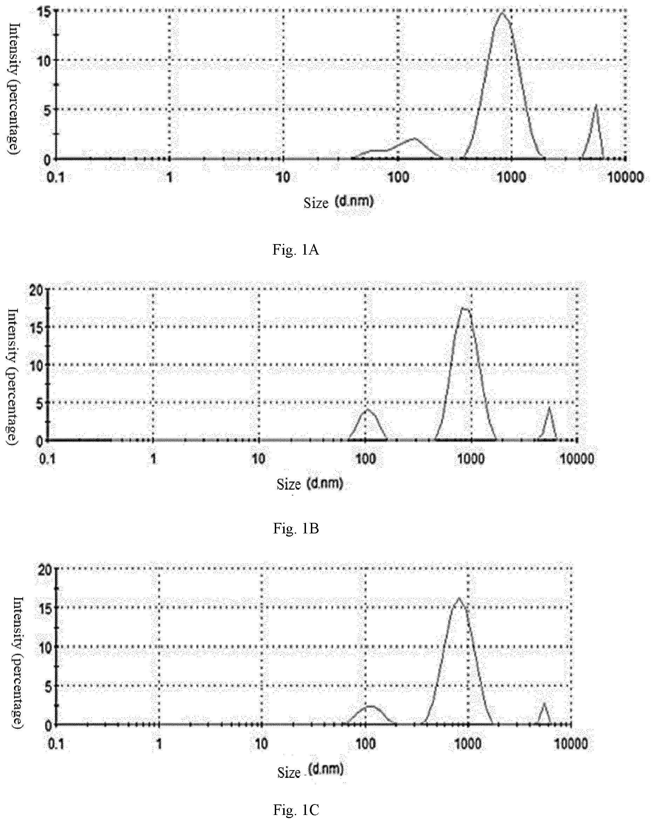

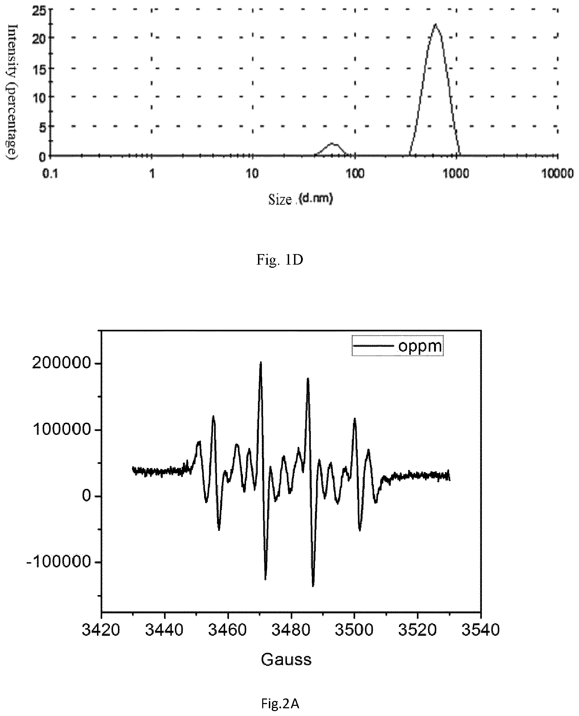

Raw materials in the table were prepared into 0.1% aqueous solutions, and particle sizes were measured by means of dynamic light scattering. Results are shown in Figure 1.

| Raw material | DLS mean particle size nm |
|---|---|
| Hydroxyethyl cellulose-fullerene | 740 |
| Sodium hyaluronate-fullerene | 730 |
| Glucose-fullerene | 670 |
| Hydroxyethyl cellulose: sodium hyaluronate: glucose-fullerene | 738 |

### Embodiment 4: Testing of stabilities of various raw materials by means of a high-speed centrifugation

Raw materials in the following table were prepared into 0.1% aqueous solutions, and the aqueous solutions were centrifuged at 4000 r/min for 15 min (referring to the General Rule 0123 of *Pharmacopoeia I*) to test stabilities of the solutions.

| Raw material | Stability |
|---|---|
| Hydroxyethyl cellulose-fullerene | No precipitate |
| Sodium hyaluronate-fullerene | Had precipitates |
| Glucose- fullerene | Had precipitates |
| Hydroxyethyl cellulose-sodium hyaluronate-glucose-fullerene | No precipitate |

### Embodiment 5: Testing of hydroxyl free radical scavenging capacities of the compositions of the present invention at different concentrations by using a spin trapping method

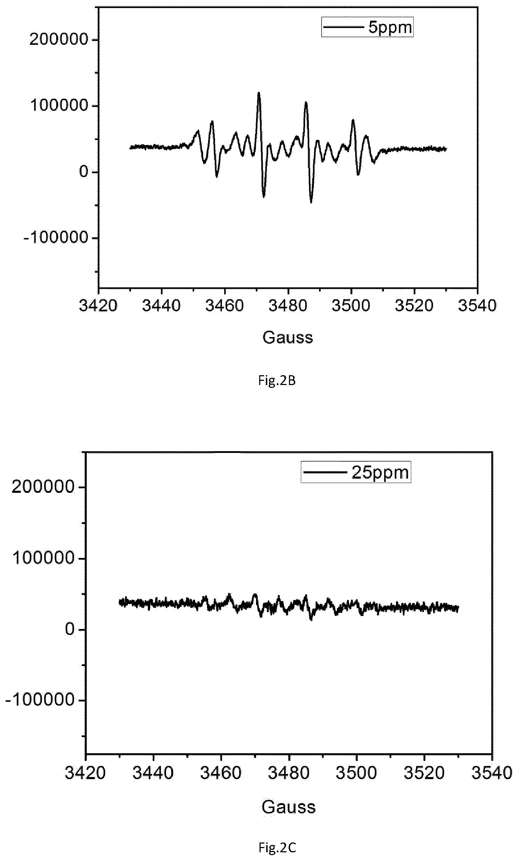

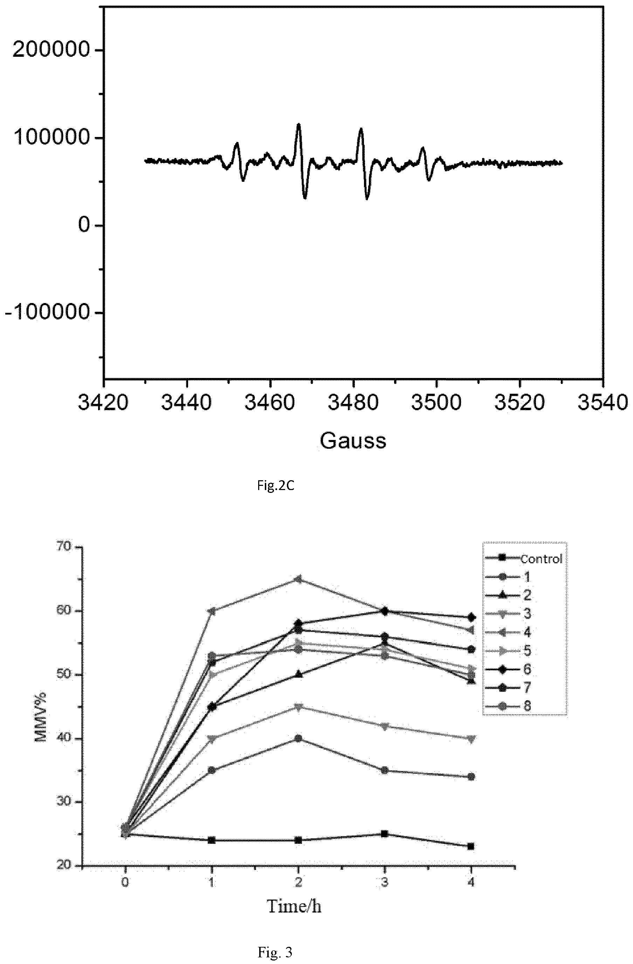

5 uL of a sample at a certain concentration was added into 20 uL of 0.1 mol/L DMPO and 20 uL of an 0.03 mol/L H₂O₂ aqueous solution, ultraviolet light excited hydrogen peroxide to produce free radicals, and a hydroxyl free radical scavenging effect of the composition was analyzed by detecting a change of an esr signal. Results are shown in figure 2. Figure 2A is a blank test, figure 2B is a 5 ppm (the content of fullerene) test group, figure 2C is a 25 ppm (the content of fullerene) test group, and figure 2D is a 25 ppm water-soluble vitamin C. It can be seen from figures 2A-2C, at the same concentration, the fullerene has a better hydroxyl free radical scavenging capacity compared with vitamin C.

### Embodiment 6: Testing of moisturizing and hydrating properties

Skin moisture content tester: Corneometer 825 Courage+Khakaza Cologne Germany.

Skin water loss tester: TM300 Courage+Khakaza Cologne Germany.

Moisturizing and moisture locking properties of raw materials are determined according to skin moisture content (MMV) values and transepidermal water loss values obtained by tests (MMV 25-35 means ordinary, 35-45 means medium, 45-55 means good, and more than 55 means excellent; TEWL 11-12 means ordinary, 12-13 means medium, 13-14 means good, and more than 14 means excellent).

### Preparation of a sample to be tested:

Raw materials in the table were prepared into aqueous solutions with mass fractions of 0.5%

| Number | Raw material | Moisturizing (excellent, good, medium, ordinary) | Water-locking (excellent, good, medium, ordinary) | Skin feeling (excellent, good, medium, ordinary) |
|---|---|---|---|---|
| 1 | Hydroxyethyl cellulose-fullerene | Ordinary | Medium | Ordinary |
| 2 | Sodium hyaluronate-fullerene | Excellent | Medium | Medium |
| 3 | Glucose-fullerene | Medium | Medium | Ordinary |
| 4 | Hydroxyethyl cellulose-sodium hyaluronate-glucose-fullerene: 30: 10: 59: 1 | Excellent | Excellent | Excellent |
| 5 | Hydroxyethyl cellulose-sodium hyaluronate-glucose-fullerene: 40: 10: 49: 1 | Medium | Medium | Medium |
| 6 | Hydroxyethyl cellulose-sodium hyaluronate-glucose-fullerene: 30: 20: 49: 1 | Good | Good | Good |
| 7 | Hydroxyethyl cellulose-sodium hyaluronate-glucose-fullerene: 20: 10: 69: 1 | Good | Medium | Ordinary |
| 8 | Hydroxyethyl cellulose-sodium hyaluronate-glucose-fullerene: 30: 5: 64: 1 | Medium | Medium | Ordinary |

Control: a blank without applying any skin care product
Skin moisture contents of insides of elbows of 30 subjects before and 1h, 2h and 4h after applying serums added with different raw materials were measured, a mean MMV value was calculated, and results are shown in Figure 3.

It can be seen from Figure 3 that the composition of the invention has a better moisturizing property, and still keeps a good moisturizing property after 4 h. An effect of the composition composed of a combination of hydroxyethyl cellulose, sodium hyaluronate and glucose and fullerene is better than that of the composition composed of each of the above raw materials and fullerene, and an effect of the composition of the present invention in a most preferably ratio was better than that in other ratios.

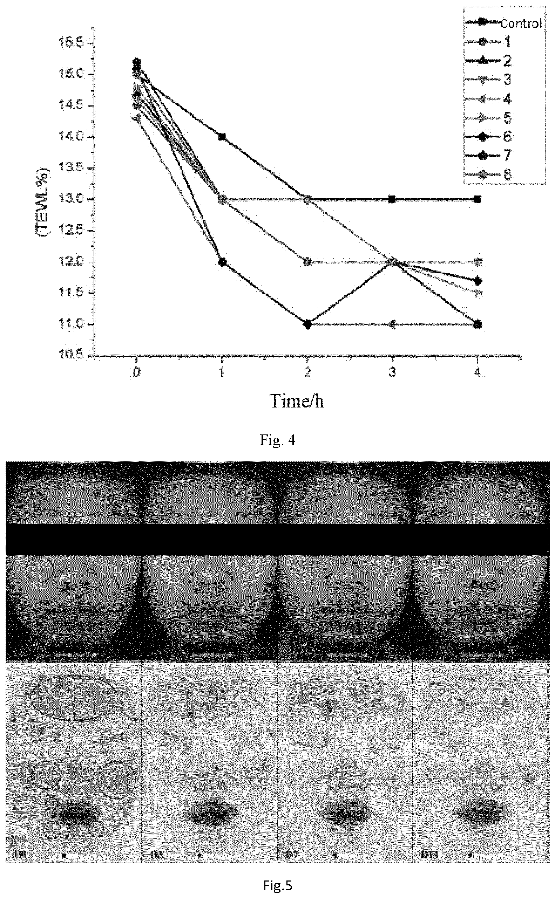

Skin water losses of the 30 subjects before and 1 h, 2 h, and 4 h after applying the serums added with different raw materials were measured, a mean TEWL value was calculated, and results are shown as in figure 4. It can be seen from figure 4 that the composition of the present invention had an advantage in holding moisture of skin.

### Embodiment 7: Acne-removing test

Clinical features of acnes of volunteers were graded according to acne pillsbury 4 and Cunliffel 12 classification methods. Subjects were divided into three groups according to the grading results, namely grade 1 (mild), grade 2 (moderate), and grade 3 (severe) acne subjects. Within a test period of 14 days, facial images of the subjects were acquired by means of a facial image analyzing system VISIA-CR, facial erythema images were obtained under a cross polarized light, degrees of inflammatory response and acne growth conditions of the subjects could be observed, and a deeper color in the image denotes a strong degree of inflammatory response of skin.

Within the test period of 14 days, images of the subjects before and after using a sample were acquired by means of VISIA-CR, and a comparison result of images of a grade 3 (severe) acne subject (No. 34) is shown in figure 5.

It can be seen from figure 5, within the test period of 14 days, after the grade 3 (severe) acne subject (No. 34) used the trial sample for 14 days, the colors of acne growth areas marked by a red cycle on a VISIA-CR natural light image and an erythema image gradually turned lighter, the area of redness decreased, inflammatory response around acnes decreased, and smaller acnes and a part of larger acnes basically disappeared, which indicates that the trial sample has skin inflammatory response relief and acne-removing effects.

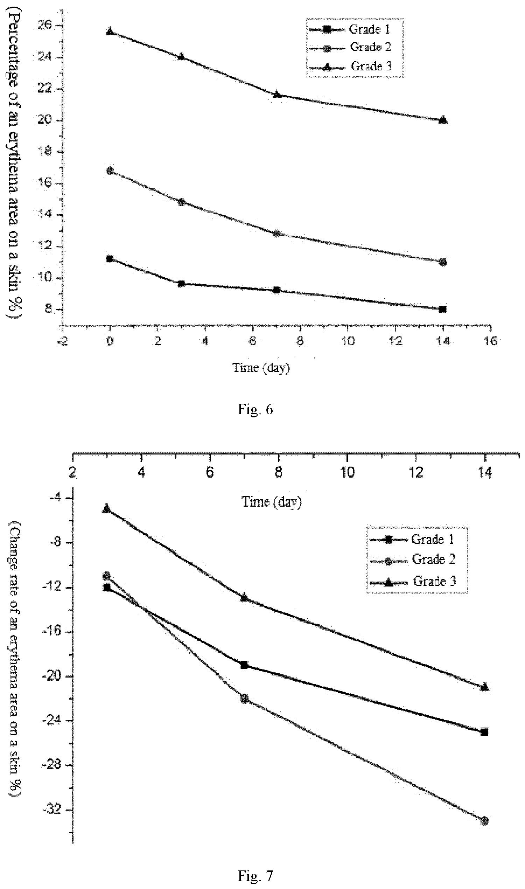

As shown in figure 6, within the test period of 14 days, after using the trial sample, percentages of the erythema areas on skins of the grade 1 (mild), grade 2 (moderate) and grade 3 (severe) acne subjects at each time points were less than those before using the trial sample, and there were significant differences (*p*< 0.05) between the percentages of the erythema areas on the skins of the grade 1 (mild) acne subjects on the 7th day and 14th day, and there were significant differences between the percentages of the erythema areas on the skins of the grade 2 (moderate) and grade 3 (severe) acne subjects (*p*< 0.05) at each time points.

As shown in Figure 7, change rates of the percentages of the erythema areas on the skins of the grade 1 (mild), grade 2 (moderate) and grade 3 (severe) acne subjects at each time points were all negative.

From the above, it is indicated that the trial sample can significantly reduce the percentage of the erythema area on a skin, and has significant effects of relieving skin inflammatory response and removing acnes.

### Embodiment 8: Whitening test

Testing method: after mice were anesthetized with 2.5% pentobarbital sodium, and hindquarter muscle of the mouse in a model group was injected with 0.4% progesterone injection at a dose of 0.02 g/kg, 6 times a week for 4 consecutive weeks. Muscle of the mouse in a normal group was injected with sterilized water for injection at a dose of 0.02 g/kg. 60 healthy female mice were randomly divided into 6 groups with 10 mice in each group. The normal group, the model group, 3 dose groups (0.1%, 0.2% and 0.3%) for external use of a fullerent composition (wherein, a mass ratio of hydroxyethyl cellulose, sodium hyaluronate, glucose and fullerene was 30: 10: 59: 1), and a positive control group using 3% hydroquinone (0.69 mg/ml). The 3 dose groups were given topical medication on the skin of the mice on the modeling day, once a day, for 30 consecutive days. The normal group and the model group were given local application of distilled water at corresponding time; the positive control group using 3% hydroquinone began topical administration on the modeling day, the time and treatment course were the same as those of the fullerene group. After administration for 30 days, the mice were killed by means of cervical dislocation, fur on back was removed, 2 pieces of each of a liver tissue and a skin tissue of the applied portion were quickly taken, wherein one piece was washed with ice-cold normal saline to remove blood and dried with a filter paper, 0.5 g of each of the liver and the skin was cut, and respectively placed into a beaker with 2.0 ml of pre-cooled normal saline, the tissue was cut into pieces and then put into a tube, homogenized with a high-speed disperser at 10 r/min twice according to 10 s/time, then centrifugated at a rotary speed of 3500 r/min, and supernatant was taken for test.

Results: see the table below

| Group | N | Skin SOD (nU/g) | Skin MDA (nmol/g) | Number of dermal blood vessels | Number of dermal inflammatory cells | Number of melanocytes |
|---|---|---|---|---|---|---|
| Normal group | 10 | 0.570 count 0.037 | 0.052 count 0.037 | 1 | 2 | 1 |
| Model group | 10 | 0.391 | 0.086 | 2 | 6 | 7 |
| | | count 0.037 | count 0.037 | | | |
| Positive group | 10 | 0.478 count 0.037 | 0.073 count 0.037 | 7 | 5 | 5 |
| 0.1% of the composition of the present invention | 10 | 0.531 count 0.037 | 0.060 count 0.037 | 8 | 3 | 5 |
| 0.2% of the composition of the present invention | 10 | 0.574 count 0.037 | 0.053 count 0.037 | 9 | 3 | 3 |
| 0.3% of the composition of the present invention | 10 | 0.612 count 0.037 | 0.047 count 0.037 | 10 | 4 | 2 |

Conclusion: as shown in the above table, the normal group is not subjected to modeling, the model group is treated with the drug after modeling, and the positive group is hydroquinone having a whitening effect known. After the success of the modeling, the number of melanocytes of model group significantly increases, the number of dermal inflammatory cells significantly increases, the number of dermal blood vessels decreases, and compared to the low, medium and high dose groups, it is found that the numbers of melanocytes and the numbers of dermal inflammatory cells of the dose groups are both less than those of the model group, but the numbers of dermal blood vessels significantly increases, which indicates that the dose groups all have a certain whitening effect, and have a certain concentration dependence. Compared to the model group and the positive control group, the composition of the present invention has a significant effect, and has whitening and acne-removing functions.

Face creams added with 2% of the composition of the present invention were given to the volunteers for trial. Inclusion criteria for volunteers:
① 33 cases recruited, 30 qualified cases, age of 35-60, both male and female;
② medical and health conditions are in accordance with the recruited criteria;
③ no allergy to cosmetics;
④ no corticosteroids, antibiotics, retinoids or other anti-acne drugs were taken 2 weeks before the treatment;
⑤ did not take laser beauty or chemical peel in the last 2 years;
⑥ no ethnic contraindication;

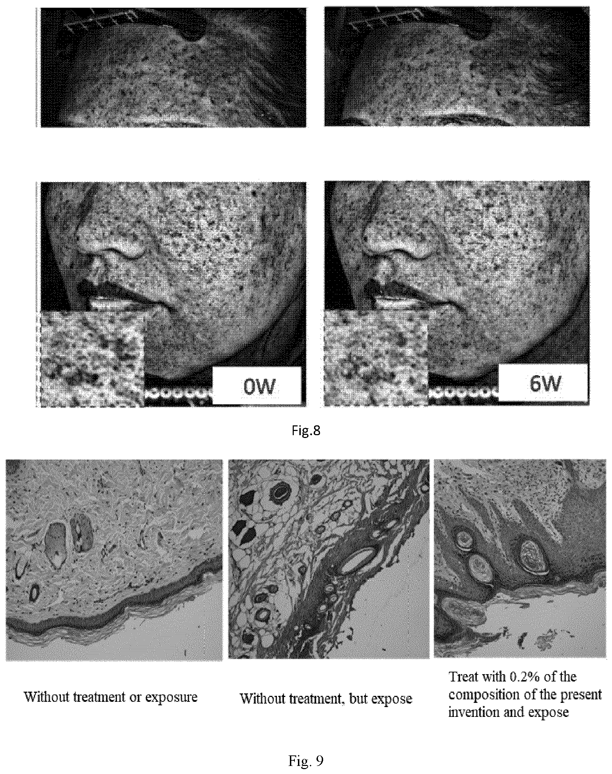

It can be seen from figure 8 that after using the product containing 0.2% of the composition of the invention for 6 weeks, melanin in the cheek area was significantly reduced, and the fairness of skin was significantly improved.

### Embodiment 9: Haze-proofing test

A skin explant was treated by using a fullerene composition of the present invention (wherein, a mass ratio of hydroxyethyl cellulose , sodium hyaluronate , glucose and fullerene was 30: 10: 59: 1) for 5 days, and then exposed to a polluted environment. Results showed that there was no significant difference between cell states of the skin treated by using the composition of the present invention and that of a blank group. However, injured cells of the skin damaged by haze significantly increased, and results are shown in figure 9.

## Claims

1. A fullerene composition, comprising a carbohydrate additive and a fullerene dissolved or dispersed in the additive, optionally, the composition being in a powder form or a solution form.

2. A fullerene dispersion system, comprising a fullerene and a carbohydrate additive, **characterized in that** more than 50%, such as more than 60%, 70%, 80%, 90%, 95%, 99%, of particle sizes in the dispersion system are between 690 nm and 700 nm.

3. A cosmetic or drug delivery and/or a sustained-release system, **characterized by** comprising the composition or the dispersion system of any one of the preceding claims.

4. A preparation method for a fullerene composition or a dispersion system, **characterized in that** the preparation method comprises the following steps: (1) uniformly mixing a fullerene powder and a carbohydrate powder; (2) grinding the mixture; (3) dissolving with water; (4) centrifuging at a high speed; and (5) filtering; optionally, the method further comprises the steps of (6) concentrating; and (7) drying; optionally, a finished product obtained at step (7) may be further dissolved with water or reconstituted as a required solution; optionally, during the grinding, a dissolution degree is monitored by means of ultraviolet absorbance; optionally, the grinding is performed by use of a ball grinder, a colloid grinder, a sand grinder or a homogenizer; and optionally, filtering is performed by using a G4 sand core funnel.

5. A fullerene composition, **characterized in that** the composition is prepared by the method of claim 4.

6. The composition or the dispersion system or the method of any one of the preceding claims, wherein the carbohydrate additive is selected from monosaccharide, disaccharide, oligosaccharide, polysaccharide and a saccharide amine; optionally, the monosaccharide is selected from fructose, galactose and glucose; optionally, the disaccharide is selected from sucrose, lactose, maltose, melibiose and cellobiose; optionally, the polysaccharide is selected form cellulose and a hyaluronic acid; optionally, the saccharide amine is selected form acetylchitosamine, lactamide and glucosamine; optionally, the cellulose is, such as, hydroxyethyl cellulose, ethyl cellulose, microcrystalline cellulose, hydroxypropyl methyl cellulose and methyl cellulose; optionally, the hyaluronic acid is, such as, hyaluronic acid, sodium hyaluronate, potassium hyaluronate, hydrolyzed hyaluronic acid and hydrolyzed sodium hyaluronate; preferably, the carbohydrate additive is at least one of hydroxyethyl cellulose, sodium hyaluronate and glucose, more preferably, the carbohydrate additive is hydroxyethyl cellulose, sodium hyaluronate and glucose; and optionally, a weight ratio of the carbohydrate additive to the fullerene is 50-200: 1.

7. The composition or the dispersion system of any one of the preceding claims, wherein the fullerene is at least one of a hollow fullerene, a metallic fullerene, a heterocyclic fullerene and an endohedral fullerene, comprises but is not limited to any one or a mixture of fullerene C₂ₙ, M@C₂ₙ, M₂@C₂ₙ, MA@C₂ₙ, M₃N@C₂ₙ, M₂C₂@C₂ₙ, M₂S@C₂ₙ, M₂O@C₂ₙ and MₓA₃₋ₓN@C₂ₙ, wherein, M and A are both metallic elements, and the M and A are both selected from any one of Sc, Y and a lanthanide metallic element; and are 00 family elements among the elements of 30 families, preferably, the fullerene is any one or a mixture of C60, C70, C76, C84, Gd@C82 and derivatives thereof, further preferably, the fullerene is C60.

8. The composition or the dispersion system of any one of the preceding claims, **characterized in that** the composition or the dispersion system is composed of hydroxyethyl cellulose, sodium hyaluronate, glucose and a fullerene, optionally, a ratio of the composition is (10-50): (5-20): (30-80) : (0.1-10), for example, the ratio is 40: 10: 49: 1, 30: 20: 49: 1, 20: 10: 69: 1, 30: 5: 64: 1, 30: 10: 59: 0.1, preferably, the ratio is 30: 10: 59: 1; and optionally, the composition further comprises other organic solvents and/or water serving as a solvent.

9. An application of the composition or the dispersion system of any one of the preceding claims in preparation of a beauty product or a cosmetic.

10. An application of the composition or dispersion system of any one of the preceding claims in manufacturing a beauty or make-up tool.
